# EUROPEAN PATENT APPLICATION

(11) **EP 0 873 986 A1**
(43) Date of publication of application: **28.10.1998**
(21) Application number: 98303024.8
(22) Date of filing: 17.04.1998
(51) Int. Cl.: C07C 215/90, C08K 5/42, C09D 7/12

(54) **Antistatic agents**

(30) Priority: 22.04.1997 JP 104868/97
(71) Applicant: ALTECH COMPANY LIMITED, Tokyo (JP)
(72) Inventor: Hayashi, Kanji, Ota-ku, Tokyo (JP)
(74) Representative: King, James Bertram

(57) **Abstract**

Antistatic agents, adhesives and coatings having high antistatic properties under low ambient humidity and temperature include the compound of formula:

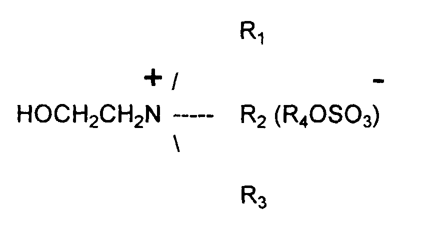

This antistatic agent is an ammonium quaternary salt with the disclosed structure. The compound is an alkyl based agent with carbon number C1∼C5 for R1, R2, and R3, and carbon number C1∼C2 for R4.

## Description

This invention relates to antistatic agents, coatings and adhesives which are used in the production of various industrial materials, such as for melt or sublimation type thermal transfer ink ribbons and laminated packaging materials. The antistatic properties of these materials are maintained without reducing their antistatic induction properties under low ambient temperature when used as adhesive primers for plastic film or as adhesives.

Antistatic materials in current use are surfactants, and the antistatic property depends largely on the ion electroconductivity generated by moisture intervention. Since the ion electroconductivity decreases under low ambient humidity, the quantity of antistatic agent used as antistatic measure must be adjusted to reflect changes in humidity, such as different humidity levels between winter and summer.

Known antistatic agents present various problems. The problem of using surfactants as antistatic agents lies in bleed effects, as large usage of surfactants interferes with the adhesive strength of the laminated film. While it is more desirable to use polymer type antistatic agents, their antistatic property also depends on the ion electroconductivity, and is susceptible to low ambient humidity. This invention seeks to solve these problems.

An object of this invention is to provide a substance possessing the property of an ion carrier preventing the decrease of ion conductivity.

In accordance with this invention there is provided an antistatic agent comprising the compound characterised by the structure: 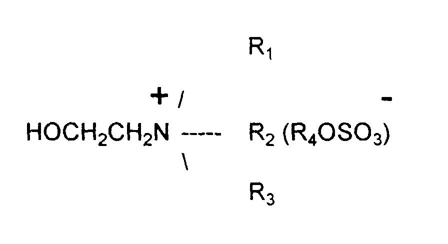 having a carbon number C1∼C5 for R1, R2, and R3, and a carbon number C1∼C2 for R4.

Preferably the antistatic agent is an ammonium quaternary salt with the aforesaid structure and may comprise an alkyl based agent with carbon number C1∼C5 for R1, R2, and R3, and a carbon number C1∼C2 for R4.

This invention also provides antistatic coatings and adhesives produced by adding the ammonium quaternary salt antistatic agent described to water soluble polymer and aqueous emulsion polymer type resinous materials.

This invention further provides antistatic coatings and adhesives produced by adding the ammonium quaternary salt antistatic agent described to polymer type resinous materials soluble in ketones, esters and alcohol solvents. This invention avoids potential problems caused by static electricity without reducing the antistatic conductivity properties of polymer resin products under low ambient humidity.

When acrylic copolymer possessing an ammonium quaternary base is used on the chemical chain as a polymer type antistatic adhesive primer, the surface resistivity of the primer under 50 to 60% ambient humidity is 10⁸ to 10¹⁰Ω/cm², reflecting a sufficient antistatic property. However, the surface resistivity increases to 10¹¹ to 10¹³Ω/cm² under 15 to 20% ambient humidity, showing a tendency to decrease the antistatic property considerably. This problem occurs particularly during periods of lower humidity such as in winter.

This decrease of antistatic property, however, is not only caused by low humidity. An increase of surface resistivity due to the free movement (migration) of the polymer in the molecular matrix can also be observed with polymer type antistatic agents exposed to low temperature conditions.

The decrease of antistatic performance under low ambient temperature and humidity can be solved by adding the ammonium quaternary salt of this invention to the above mentioned adhesive primer compounds. This method can be applied not only to the adhesive primer compounds but also to the other adhesive compounds mentioned giving similar effects.

An effective antistatic performance of the plastic laminated film under low ambient humidity can be obtained using the adhesive agents reacting with polyisocyanate through the addition of the ammonium quaternary salt to the polyether polyol resinous solvent. this is especially true with urethane type adhesives.

The chemical compound of this invention can be added to the binder of a printing ink or to the medium or component for improving the antistatic performance of the printing ink.

Combining the acrylic acid ester (including meta) of the compound of this invention with an acrylic copolymer creates a highly heat resistant antistatic polymer. Heat resistant antistatic coatings capable of retaining an antistatic quality under exposure to 150° C temperature for a period of 60 minutes, can be created with the combined components of this polymer and epoxy compound. However this polymer is basically ion electroconductive also, and the antistatic property decreases under low ambient humidity. In this case, the decrease of antistatic performance under low ambient humidity can be prevented by adding the chemical compound of the invention to the above polymer components.

The ammonium quaternary salt with the Chemical Structure of this invention can form quaternary compounds through alkylsulfonation of dimethylaminoethanol with dialkyl sulfate. The quaternary compounds of this invention are compounds with a Chemical Structure (2) and a Chemical Structure (3) consisting of dimethylsulfate or diethylsulfate.

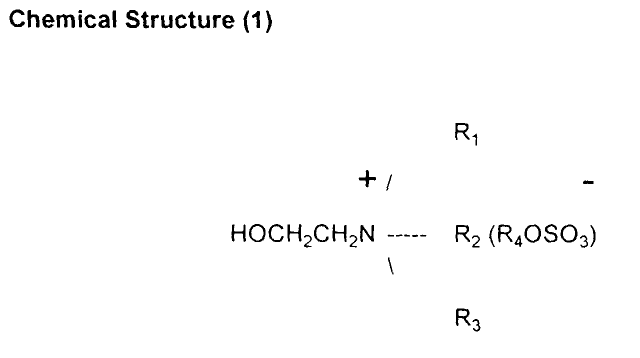

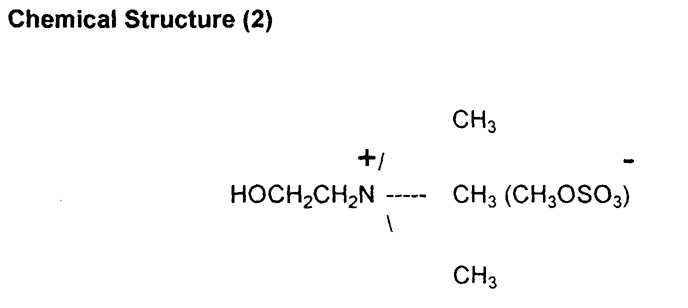

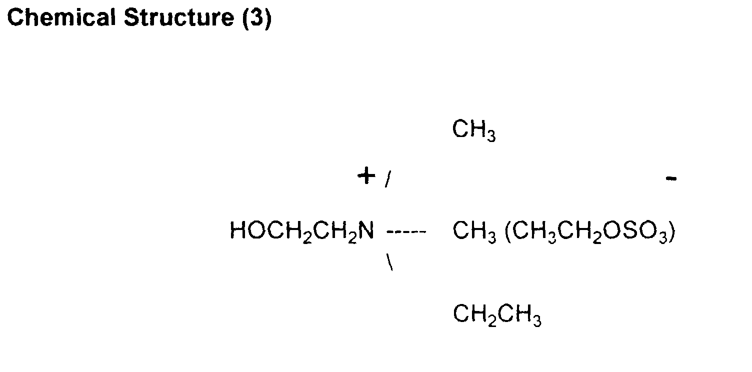

The acrylic ester (including meta) of these quaternary compounds is effective as a comonomer of antistatic acrylic copolymers, and their copolymers are extremely useful as main components of the above described heat resistant antistatic adhesive primer. Addition of Chemical Structure (3) to this adhesive primer is especially effective against low humidity.

Furthermore, antistatic adhesive agents for dry laminates, previously unavailable, can now be achieved by reacting the terminal of this quaternary compound with the OH base and isocyanate NCO base. The structure and properties are explained hereunder using examples.

### [Example 1]

Coatings with excellent antistatic and heat resistant properties were achieved with components whose main constituent is a solution of IPA/water base solvent. This coating material is an acrylic copolymer consisting of metacrylate of Chemical Structure (2)/methyl metacrylate (MMA)/ethyl acrylate (EA)/ acrylic acid (AA), added to polyfunctional epoxy compounds as crosslinking agents and polyethyleneimine as a crosslinking catalyst. After the above coatings were applied, dried, and hardened on the surface treated PET film, the surface resistivity under normal temperature and after heating were measured.
The measured values are as follows:

### (Surface Resistivity).

1. Under normal temperature: 3∼5 x 10⁹Ω/cm²
2. After heating for 60 minutes at 150°C: 4∼6 x 10⁹Ω/cm²

- Measuring environment:: Temperature = 20°C, Humidity = 50% RH

### [Example 2]

Surface resistivity under 20°C temperature and 20% humidity was measured after adding 10% of Chemical Structure (3) compound to the solid polymer matter of the coating compounds of Example 1.

### (Surface Resistivity)

1. Coated product as per example 2: 5.5 x 10⁹Ω/cm²
2. Coated product as per example 1: 6.5 x 10⁹Ω/cm²

### [Example 3]

The laminated film created by combining OPP film (20 microns) and PE (30 microns) using the compounds which combine isocyanate type urethane adhesives to the Chemical Structure (3) components showed low tribostatic volts, achieving an excellent antistatic film. Formulations of the adhesives are shown in Table 1 and Table 2.

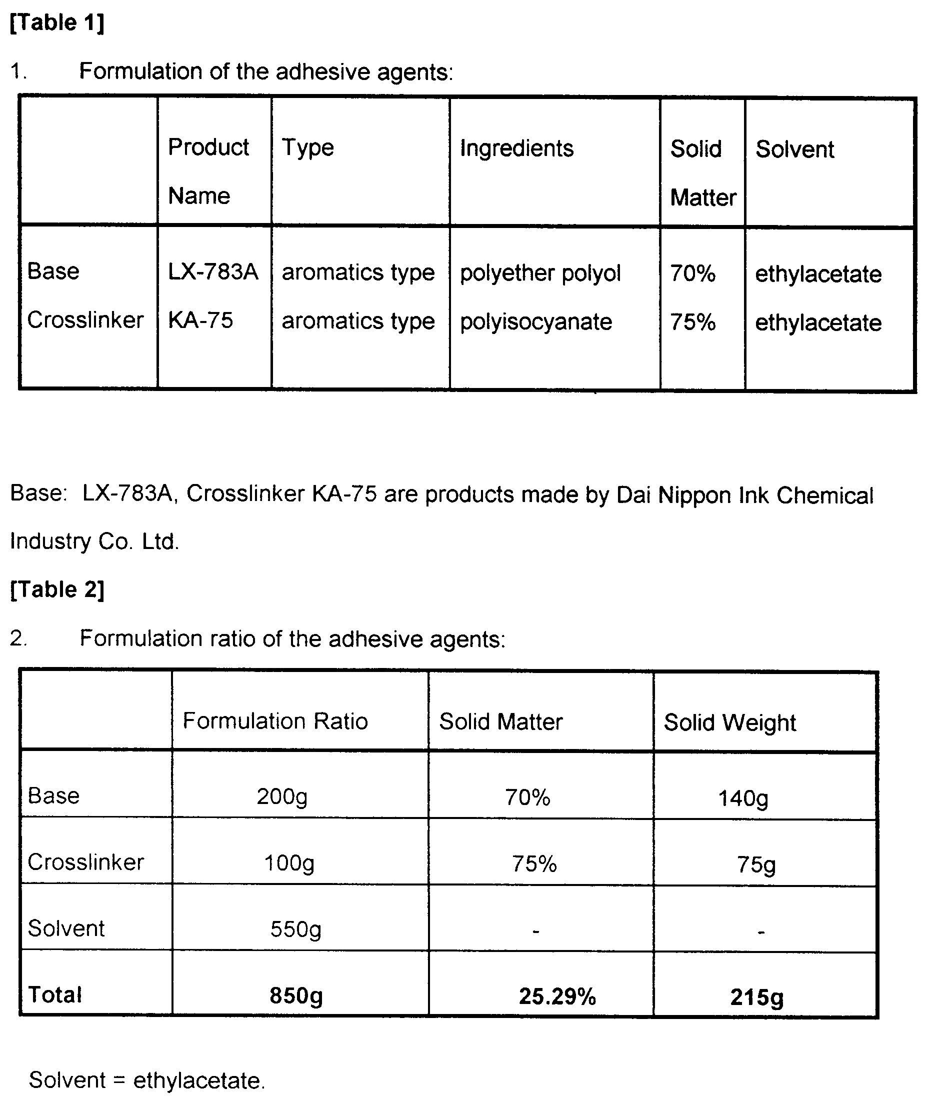

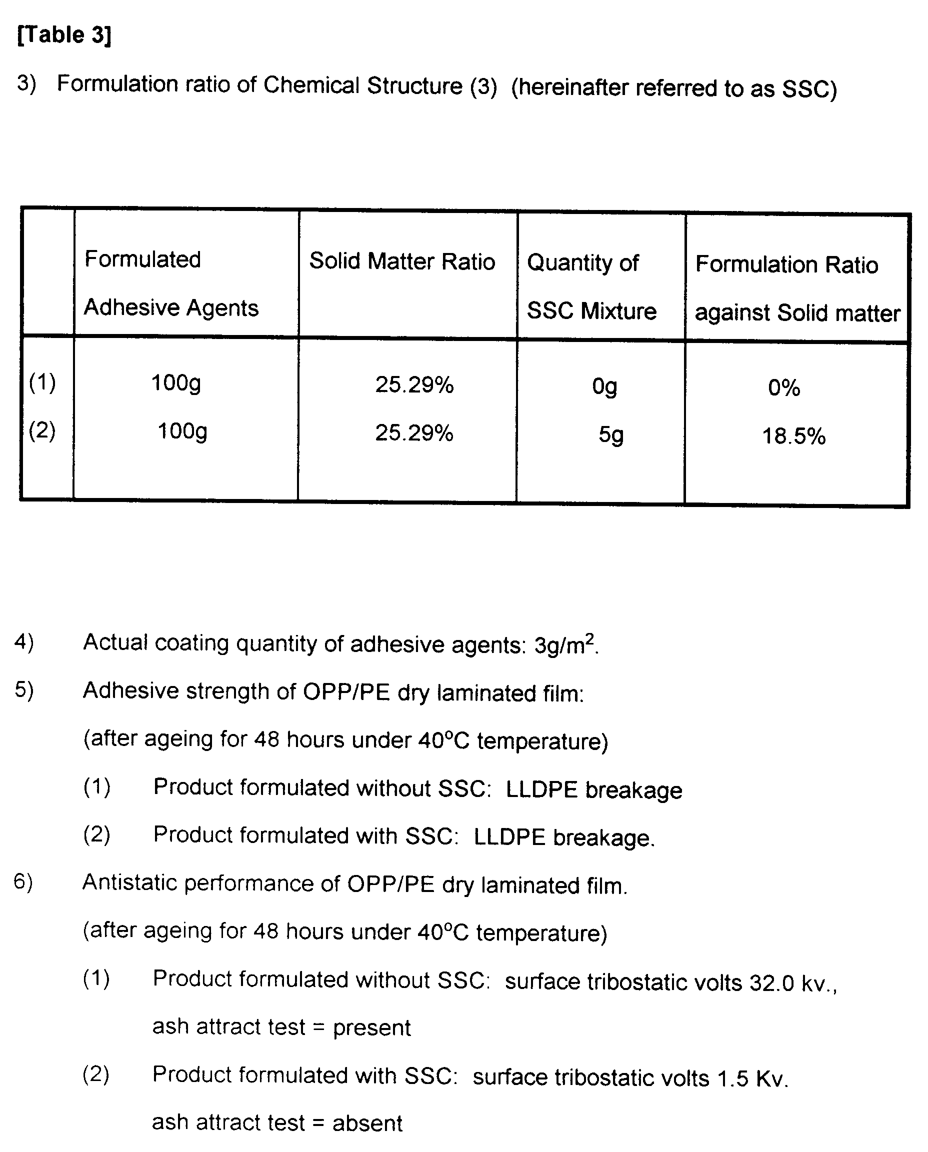

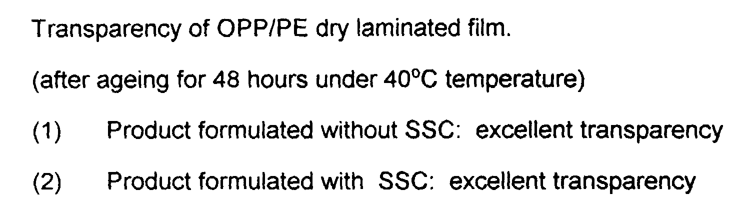

The antistatic agents proposed by this invention possess excellent antistatic performance under low ambient humidity and temperature, and are highly effective in preventing static problems with various polymer resin products under such environmental conditions. This invention is especially effective in preventing static problems with sublimation type thermal transfer ink ribbons.
Amongst other applications are:
1. Antistatic agents for urethane type ink binders.
2. Antistatic agents for urethane type paint binders.
3. Antistatic agents for waterproof overcoatings.
4. Antistatic adhesive primer for soft PVC.
5. Antistatic agents for water based PS ink medium.
6. Modifier for obtaining antistatic isocyanate compounds.
7. Antistatic urethane type AC agents.

## Claims

1. An antistatic agent comprising the compound characterised by the structure: 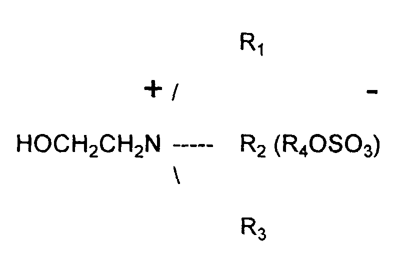 having a carbon number C1∼C5 for R1, R2, and R3, and a carbon number C1∼C2 for R4.

2. An antistatic agent in accordance with Claim 1, characterised by the structure:

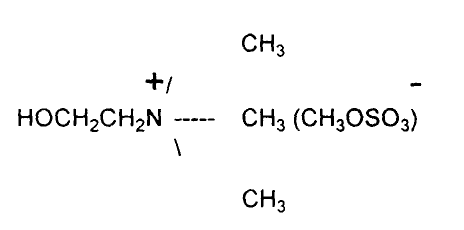

3. An antistatic agent in accordance with Claim 1, characterised by the structure:

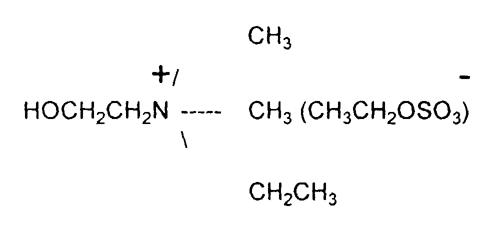

4. Antistatic coatings or adhesives produced by adding an ammonium quaternary salt according to Claim 1 or 2 or 3 to water soluble polymer or aqueous emulsion polymer resinous materials.

5. Antistatic coatings or adhesives produced by adding an ammonium quaternary salt according to Claim 1 or 2 or 3 to polymer resinous materials soluble in ketones, esters and alcohol solvents.

6. In combination the antistatic agent of Claim 1, 2 or 3, and a printing ink or printing ink base, preferably urethane based, and more preferably a sublimation transfer thermal ink ribbon.

7. In combination the acrylic acid ester of the antistatic agent of Claim 1 and an acrylic copolymer forming a heat resistant antistatic polymer coating.

8. An antistatic heat resistant coating material, comprising an acrylic copolymer consisting of the metacrylate of the structure of Claim 2, added to polyfunctional epoxy compounds as crosslinking agents and polyethyleneimine as a crosslinking catalyst.

9. An antistatic heat resistant plastic laminated film, formed by reacting the adhesive agents of Claim 5 with polyisocyanate through the addition of the ammonium quaternary salt to the polyether polyol resinous solvent.
